(19) Europäisches Patentamt
European Patent Office
Office européen des brevets

(11) Numéro de publication: **0 055 958**
**B1**

# (12) FASCICULE DE BREVET EUROPEEN

(45) Date de publication du fascicule du brevet: **25.04.84**

(21) Numéro de dépôt: **81402027.7**

(22) Date de dépôt: **18.12.81**

(51) Int. Cl.³: **B 01 J 23/26**, B 01 J 27/18, B 01 J 35/10, C 07 C 19/08, C 07 C 17/20

(54) **Catalyseurs de fluoruration en phase gazeuse des hydrocarbures chlorés et chlorofluorés aliphatiques, à base de sels ou oxydes de chrome et de phosphate d'aluminium, et procédés de fluoruration utilisant ces catalyseurs.**

(30) Priorité: **29.12.80 FR 8027662**

(43) Date de publication de la demande: **14.07.82 Bulletin 82/28**

(45) Mention de la délivrance du brevet: **25.04.84 Bulletin 84/17**

(84) Etats contractants désignés: **BE DE FR GB IT NL**

(56) Documents cités:
**GB - A - 943 627**
**US - A - 3 600 450**
**US - A - 3 862 964**

(73) Titulaire: **Société ATOCHEM, 12-16, Allée des Vosges, F-92400 Courbevoie (FR)**

(72) Inventeur: **Foulletier, Louis, 5, chemin Croix-Berthet, F-69600 Oullins (FR)**

(74) Mandataire: **Rochet, Michel et al, ATOCHEM Service PROPRIETE INDUSTRIELLE Tour Manhattan Cédex 21, F-92091 Paris la Défense (FR)**

EP 0 055 958 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

# Catalyseurs de fluoruration en phase gazeuse des hydrocarbures chlorés et chlorofluorés aliphatiques, à base de sels ou oxydes de chrome et de phosphate d'aluminium, et procédés de fluoruration utilisant ces catalyseurs

La présente invention concerne des catalyseurs de fluoruration en phase gazeuse par l'acide fluorhydrique anhydre des hydrocarbures chlorés ou chlorofluorés aliphatiques, constitués par des sels ou oxydes de chrome associés au phosphate d'aluminium. Elle concerne également des procédés de fluoruration de ces dérivés chlorés ou chlorofluorés utilisant ces catalyseurs dans des réacteurs à lit fluidisé.

De très nombreux catalyseurs ont été proposés pour ces réactions de substitution d'atomes de chlore par des atomes de fluor, en phase gazeuse. Ce sont le plus souvent des oxydes ou des halogénures de chrome, d'aluminium, de cobalt, de fer, de titane, de nickel, de cuivre, de palladium ou de zirconium, utilisés tels quels ou sur des supports divers.

C'est ainsi que le brevet français n° 720 474 et son certificat d'addition n° 43 972 enseignent la fluoruration en phase gazeuse d'hydrocarbures contenant un halogène autre que le fluor sur des catalyseurs à base de charbon de bois, imprégné ou non d'halogénures métalliques autres que les halogénures d'antimoine.

Le brevet britannique n° 2 210 369 revendique la fluoruration d'halohydrocarbures de $C_1$ à $C_3$ sur des catalyseurs à base d'halogénures de chrome déposés sur coke ou sur charbon actif.

Le brevet français n° 2 000 688 préconise l'emploi, comme catalyseur de la réaction du chlore et de l'acide fluorhydrique avec le tétrachloréthylène, du trifluorure de chrome supporté sur charbon de bois, coke de pétrole ou coke de houille.

Le brevet britannique n° 896 068 et le brevet des Etats-Unis n° 3 157 107 décrivent la préparation de catalyseurs à base d'oxyde de chrome déposé sur alumine activée.

Le brevet des Etats-Unis n° 3 992 325 révèle l'utilisation d'hydroxyde-oxyde de chrome $\gamma$-CrOOH déposé sur des fluorures minéraux, comme les fluorures alcalino-terreux.

Le brevet des Etats-Unis n° 2 755 313 décrit l'utilisation, dans la réaction de fluoruration de l'hexachlorétane formé in situ, de trifluorure d'aluminium, préparé à partir de trichlorure d'aluminium gazeux ou liquide.

Tous ces catalyseurs de l'art antérieur conviennent plus ou moins bien à la fluoruration en phase gazeuse des chloroalcanes et des chlorofluoroalcanes dans des réacteurs à lit fixe. Ils sont par contre très mal adaptés à la fluoruration dans des réacteurs à lit fluidisé, qui exigent des particules de forme régulière et de granulométrie homogène. Le simple broyage du catalyseur, suivi d'un tamisage pour sélectionner les particules de taille convenable, donne des grains de forme irrégulière et conduit à des déchets importants de catalyseur.

La demanderesse a découvert que les catalyseurs de fluoruration en phase gazeuse s'empoisonnent principalement par la formation de goudrons à leur surface. L'emploi de réacteurs à lit fluidisé, provoquant l'abrasion des grains de catalyseur, permet d'éliminer ces goudrons et de maintenir l'activité du catalyseur. La fluidisation procure également une bonne homogénéité de la température de fonctionnement du catalyseur et un contrôle très de cette température, ce qui augmente encore la durée de vie du catalyseur et réduit la formation indésirée de produits de craquage.

Les catalyseurs de l'art antérieur présentent également au moins l'un des inconvénients suivants:

— faible taux de transformation de l'acide fluorhydrique
— faible productivité
— faible sélectivité
— taux élevé d'isomères asymétriques dans la production du trichlorotrifluoréthane et du dichlorotétrafluoréthane.

Les catalyseurs de fluoruration connus, surtout ceux contenant de l'aluminium, catalysent en effet des réactions de dismutation, comme:

$$2\ CF_2Cl-CFCl_2 \rightarrow CF_2Cl-CCl_3+CF_3-CFCl_2$$

ou d'isomérisation comme:

$$CF_2Cl-CFCl_2 \rightarrow CF_3-CCl_3$$

Les isomères asymétriques du trichlorotrifluoréthane ($CF_3-CCl_3$) et du dichlorotétrafluoréthane ($CFCl_2-CF_3$) sont plus réactifs, et par suite plus instables que les dérivés symétriques ($CCF_2Cl-CFCl_2$ et $CClF_2-CClF_2$) ce qui les rend indésirables dans beaucoup d'applications.

Selon l'invention on remédie à tous ces inconvénients en préparant des catalyseurs de fluoruration en phase gazeuse constitués de sels ou d'oxydes de chrome associés au phosphate d'aluminium, faciles à mettre en forme et présentant une mésoporosité et une macroporosité élevées.

La mésoporosité de ces catalyseurs, définie par la surface des pores dont le rayon est compris entre

4 et 5 nm, doit être supérieure à 5 $m^2/g$.

La macroporosité, définie par la surface des pores dont le rayon est égal ou supérieur à 25 nm, doit être supérieure à 2 $m^2/g$.

La surface spécifique totale des catalyseurs doit être supérieure à 200 $m^2/g$.

Ces catalyseurs permettent de s'affranchir des supports comme les charbons actifs, dont les caractéristiques varient souvent d'un lot à l'autre. Ils peuvent être obtenus à partir de matières synthétiques avec des caractéristiques parfaitement reproductibles. Un autre avantage est qu'ils peuvent être mis facilement sous une forme convenable pour leur utilisation en lit fluidisé par extrusion et granulation.

Le phosphate d'aluminium a déjà lui-même une faible activité catalytique dans les réactions de fluoruration en phase gazeuse, mais cette activité est considérablement augmentée lorsqu'il est associé à des sels ou oxydes de chrome. La teneur en chrome du catalyseur doit être de 0,1 à 3 atomes-g par litre.

Alors que les catalyseurs de l'art antérieur contenant de l'aluminium ont une forte activité isomérisante vis-à-vis du trichlorotrifluoréthane et du dichlorotétrafluoréthane, et conduisent à la formation de proportions très élevées d'isomères asymétriques de ces deux composés, les catalyseurs selon l'invention conduisent, d'une manière tout-à-fait inattendue, à la formation préférentielle d'isomères symétriques.

Ces catalyseurs peuvent être facilement obtenus en mélangeant en solution aqueuse un sel de chrome, un sel d'aluminium, de l'acide phosphorique et de l'ammoniaque. Il se forme probablement dans ces conditions un phosphate mixte de chrome et d'aluminium, qui précipite. Après lavage, le précipité peut facilement être extrudé et granulé.

Selon une variante de préparation, on forme d'abord un phosphate acide d'aluminium, que l'on met en forme par extrusion-granulation, et que l'on imprègne par une solution aqueuse de trioxyde de chrome. On réduit ensuite le chrome à l'état trivalent par un alcool, comme le méthanol.

Les exemples suivants, donnés à titre non limitatif, illustrent divers modes de préparation des catalyseurs selon l'invention et leur utilisation dans diverses réactions de fluoruration en phase gazeuse.

## Exemple 1

On coule 218 $cm^3$ d'une solution aqueuse d'ammoniaque 11 N dans 1100 $cm^3$ d'une solution de 0,6 mole de chlorure d'aluminium et de 0,63 mole d'acide phosphorique, maintenue entre 0 et 15° C.

Dans le mélange ainsi obtenu on coule sous forte agitation une solution de 0,045 mole de sulfate de chrome dans 300 $cm^3$ d'eau.

On obtient un précipité, qui est lavé deux fois avec un litre d'eau, puis avec un litre d'isopropanol.

Le produit est passé dans une extrudeuse-granulatrice, qui permet d'obtenir des petits bâtonnets, puis séché sous vide et calciné 4 heures à 400° C.

Le catalyseur obtenu présente les caractéristiques suivantes:

| | |
|---|---|
| densité | 0,687 |
| surface spécifique totale | 217 $m^2/g$ |
| surface des pores $\geq$ 25 nm | 2,47 $m^2/g$ |
| surface des pores 25 – 5 nm | 92 $m^2/g$ |
| surface des pores 5 – 4 nm | 31 $m^2/g$ |

Sur ce catalyseur, porté à la température de 405° C dans un réacteur à lit fluidisé, on fait passer un mélange d'acide fluorhydrique et de trichlorotrifluoréthane dans le rapport molaire 0,97/1, à la vitesse de 14,2 moles/h/l.

Le taux de transformation de l'acide fluorhydrique est de 82%. Les taux de transformation du trichlorotrifluorétane sont de:

74% en dichlorotétrafluoréthane, contenant 71% d'isomère symétrique
8% en monochloropentafluoréthane.

Exemple 2

(Exemple comparatif, avec le phosphate d'aluminium seul)

On opère comme dans l'exemple 1, mais en omettant l'addition du sulfate de chrome. Les caractéristiques du produit obtenu sont les suivantes:

| | |
|---|---|
| densité | 0,28 |
| surface spécifique totale | 108,2 $m^2/g$ |
| surface des pores $\geq$ 25 nm | 17 $m^2/g$ |
| surface des pores 25–5 nm | 34 $m^2/g$ |
| surface des pores 5–4 nm | 27 $m^2/g$ |

Ce catalyseur est utilisé pour la fluoruration du trichlorotrifluoréthane dans les conditions de l'exemple 1.
Le taux de transformation de l'acide fluorhydrique n'est que de 56%.
Les taux de transformation du trichlorotrifluoréthane sont de:

65% en dichlorotétrafluoréthane, contenant 5% d'isomère symétrique
1% en monochloropentafluoréthane

Exemple 3

On imprègne 150 $cm^3$ du catalyseur de l'exemple 2 avec une solution de 0,00906 mole de trioxyde de chrome $CrO_3$ dans 60 $cm^3$ d'eau et on réduit le trioxyde de chrome au méthanol. Le produit est séché à l'air chaud à 150° C.
Sur ce catalyseur on fait passer dans un réacteur à lit fluidisé, à la température de 411° C, un mélange d'acide fluorhydrique et de trichlorotrifluoéthane dans le rapport molaire 1,26/1, à la vitesse de 15,4 moles/h/l.
Le taux de transformation de l'acide fluorhydrique est de 67%.
Les taux de transformation du trichlorotrifluoréthane sont de:

70% en dichlorotétrafluoréthane, contenant 47% d'isomère symétrique
8,3% en monochloropentafluoréthane.

**Revendications**

1. Catalyseurs de fluoruration en phase gazeuse des hydrocarbures chlorés ou chlorofluorés aliphatiques caractérisés en ce que:

a) ils sont constitués de sels ou oxydes de chrome associés à du phosphate d'aluminium;
b) ils présentent une surface spécifique totale supérieure à 200 $m^2/g$, une surface des pores de rayon 4–5 nm supérieure à 5 $m^2/g$ et une surface des pores de rayon égal ou supérieur à 25 nm supérieure à 2 $m^2/g$;
c) ils contiennent de 0,1 à 3 atomes-grammes de chrome par litre.

2. Catalyseurs selon la revendication 1, caractérisés en ce qu'ils sont mis en forme par extrusion-granulation.
3. Procédé de fluoruration en phase gazeuse des hydrocarbures chlorés ou chlorofluorés aliphatiques, caractérisé par l'utilisation dans un réacteur à lit fluidisé d'un catalyseur selon l'une des revendications 1 ou 2.

**Patentansprüche**

1. Katalysatoren zur Fluorierung von aliphatischen Chlor- oder Chlorfluor-Kohlenwasserstoffen in der Gasphase, dadurch gekennzeichnet, daß sie

a) aus Salzen oder Oxiden des Chroms in Verbindung mit Aluminiumphosphat bestehen;
b) eine gesamte spezifische Oberfläche über 200 $m^2/g$, eine Oberfläche der Poren mit einem Radius von 4–5 nm über 5 $m^2/g$ und eine Oberfläche der Poren mit einem Radius gleich oder größer als 25 nm über 2 $m^2/g$ aufweisen;
c) 0,1 bis 0,3 Grammatom Chrom pro Liter enthalten.

2. Katalysatoren nach Anspruch 1, dadurch gekennzeichnet, daß ihre Formgebung durch Extrusions-Granulierung erfolgt.

3. Verfahren zur Fluorierung in der Gasphase von aliphatischen Chlor- oder Chlorfluor-Kohlenwasserstoffen, gekennzeichnet durch die Verwendung eines Katalysators nach Anspruch 1 oder 2 in einem Fließbettreaktor.

**Claims**

1. Catalysts for the gas phase fluorination of aliphatic chlorinated or chlorofluorinated hydrocarbons, characterised in that:

a) they consist of salts or oxides of chromium in association with aluminium phosphate;
b) they have a total specific surface area greater than 200 $m^2/g$, a surface area of the pores of radius 4–5 nm which is greater than 5 $m^2/g$ and a surface area of the pores of radius equal to or greater than 25 nm which is greater than 2 $m^2/g$;
c) they contain from 0.1 to 3 gram-atoms of chromium per litre.

2. Catalysts according to Claim 1, characterised in that they are shaped by extrusion granulation.

3. Process for the gas phase fluorination of aliphatic chlorinated or chlorofluorinated hydrocarbons, characterised in that a catalyst according to one of Claims 1 and 2 is used in a fluidised bed reactor.